# EUROPEAN PATENT APPLICATION

(11) **EP 2 597 151 A2**
(43) Date of publication of application: **29.05.2013**
(21) Application number: 11809093.5
(22) Date of filing: 15.07.2011
(51) Int. Cl.: C12N 1/21, C12N 15/63, C07K 14/195, A61K 39/02, C07K 16/12, G01N 33/535, C12R 1/42, A61P 31/04

(54) **RECOMBINANT MICROORGANISMS, METHODS FOR PREPARING VACCINE STRAINS, ANTIGENS, AND VECTOR VACCINE COMPOSITIONS OF SAME, USES THEREOF, AND RELATED ANTIBODIES, DIAGNOSTIC KIT, AND TREATMENT AND/OR PROPHYLACTIC METHODS**

(30) Priority: 22.07.2010 BR PI1003750
(71) Applicant: Ebert Seixas, Hanna, CEP: 14010-090 Ribeirão Preto (BR); INVENT BIOTECNOLOGIA LTDA (BR/SP), 14040-900 Ribeirão Preto, SP (BR); UNIVERSIDADE DE SÃO PAULO - USP (BR/SP), 14049-900, Ribeirão Preto, SP (BR)
(72) Inventor: EBERT SEIXAS, Hanna, 14010-090 Ribeirão Preto, SP (BR); ROQUE ANTUNES BARREIRA, Maria Cristina, 14040-130 Ribeirão Preto, SP (BR); DOS SANTOS FERRAZ, Luiz Eduardo, 37540-000 Santa Rita do Sapucai, MG (BR); DE OLIVEIRA PEREIRA, Aline Ferreira, 14030-390 Ribeirão Preto, SP (BR); FERRAZ COLBACHINI, Luciana, 37540-000 Santa Rita do Sapucai, MG (BR); RUSCA CORREO PORTO, Ana Carolina, 04514-103 São Paulo, SP (BR); ALMEIDA CARDOSO, Silvia, 36570-000 Viçosa, MG (BR); PAIVA, Helder Henrique, 14051-100 Ribeirão Preto, SP (BR); GOMES SOARES, Sandro, 14050-105 Ribeirão Preto, SP (BR); PEREIRA RUAS, Luciana, 14030-000 Ribeirão Preto, SP (BR)
(74) Representative: KATZAROV S.A.
(86) International application number: PCT/BR2011/000233
(87) International publication number: WO 2012/009774

(57) **Abstract**

The present invention provides live recombinant microorganisms such as prokaryote organisms, particularly enterobacteria, preferably *Salmonella enterica,* containing SEQ. ID. no. 1 and SEQ. ID. no. 2 capable of expressing VapG and/or VapA/VapG lipoproteins (SEQ. ID. no. 3 and SEQ. ID. no. 4), optionally in combination with other active ingredients, methods for preparing vaccine strains, antigens (SEQ. ID. no. 3 and SEQ. ID. no. 4) and vaccine compositions, preferably vectored vaccines. Additionally, the present patent application relates to the use of the vaccine vectors in the preparation of pharmaceutical compositions, particularly vaccines indicated for the prevention and/or treatment of infections by *Rhodococcus equi,* its antibodies and/or antisera, diagnostic kits and methods for the prophylaxis and/or treatment of infections by *R. equi.*

## Description

### FIELD OF THE INVENTION

The present invention provides live recombinant microorganisms such as prokaryote organisms, particularly enterobacteria, preferably *Salmonella enterica,* containing SEQ. ID. no. 1 and SEQ. ID. no. 2 capable of expressing VapG and/or VapA/VapG lipoproteins (SEQ. ID. no. 3 and SEQ. ID. no. 4), optionally in combination with other active ingredients, methods for preparing vaccine strains, antigens (SEQ. ID. no. 3 and SEQ. ID. no. 4) and vaccine compositions, preferably vectored vaccines. Additionally, the present patent application relates to the use of the vaccine vectors in the preparation of pharmaceutical compositions, particularly vaccines indicated for the prevention and/or treatment of infections by *Rhodococcus equi,* its antibodies and/or antisera, diagnostic kits and methods for the prophylaxis and/or treatment of infections by *R. equi.*

### BACKGROUND OF THE INVENTION

The genus *Rhodococcus* is composed of bacteria with varied morphologies (pleomorphic) which are Gram-positive, non-motile and found in different environments such as in the soil, the water table, oceans, intestinal microbiota of insects and surfaces of plants. All of the species from this genus are aerobic, do not sporulate, and comprise a group of bacteria with varied genetic and physiological characteristics (MARTÍNKOVÁ, L., UHNÁKOVÁ, B., PÁTEK, M., NESVERA, J., KREN, V. Biodegradation potential of the genus Rhodococcus. Environment International 2009 Jan;35(1):162-77. Epub 2008 Sep 11). Species of *Rhodococcus* have been described with potential for use in industrial and biotechnical processes due to the production of a series of enzymes of commercial interest (BELL, K. S., PHILP, J. C., AW, D. W., CHRISTOFI, N. The genus Rhodococcus. Journal of Applied Microbiology 1998 Aug;85(2):195-210). One interesting fact is that strains of *Rhodococcus* can be used in the degradation of organic compounds as a proposed bioremediation, both environmentally and industrially (GAKHAR, L., MALIK, Z. A., ALLEN, C. C, LIPSCOMB, D. A., LARKIN, M. J., RAMASWAMY, S. Structure and increased thermostability of Rhodococcus sp. naphthalene 1,2-dioxygenase. Journal of Bacteriology 2005 Nov;187(21):7222-31; PETRUSMA, M., DIJKHUIZEN, L., VAN DER GEIZE, R. Rhodococcus rhodochrous DSM 43269 3-ketosteroid 9-alpha-hydroxylase, a two-component iron-sulfur-containing monooxygenase with subtle steroid substrate specificity. Applied Environmental Microbiology 2009 Aug;75(16):5300-7. Epub 2009 Jun 26).

Some species, such as *R. bronchialis,* are occasionally related to pathologies in humans; however, the most important species as a potential pathogen is *R. equi* (VON BARGEN, K., HAAS, A. Molecular and infection biology of the horse pathogen Rhodococcus equi. FEMS Microbiology Reviews. 2009 Sep;33(5):870-91. Epub 2009 Apr 23). *R. equi* are generally found in dry soils and are dispersed in dust. This microorganism is commonly associated with illnesses in horses and is one of the main etiological agents of pneumonia in foals.

Recent data have shown that these pathogens can cause disease in pigs, cattle and goats, and they are described as attacking humans with immunodeficiencies as a comorbidity in patients infected with the human immunodeficiency virus (HIV). In humans, infections with *R. equi* are frequently associated with diseases with signs very similar to pulmonary tuberculosis and are related, however, to various extra-pulmonary pathologies. This pathogen was first isolated in 1923 in the lungs of foals with pyogranulomatous pneumonia and was classified as *Corynebacterium equi* (MAGNUSSON, H. Spezifische infektiöse Pneumonie beim Fohlen. Ein neuer Eitererreger beim Pferde [Specific infectious pneumonia in foals. A new pyogenic organism in horses]. Archive Wissen Praktise Tierheilk.1 1923;50:22-38), later called *Rhodococcus equi* (GOODFELLOW, M., ALDERSON, G. The actinomycete genus Rhodococcus: a home for the "rhodochrous" complex. Journal of General Microbiology. 1977 May;100(1):99-122).

The primary characteristics of the virulence of *R. equi* are associated with the extra-chromosomal production of antigens. These antigens are produced from plasmids, circular molecules comprised of double-stranded DNA that are capable of reproducing independently of the chromosomal DNA of the microorganism. Of particular interest among the plasmids of *R. equi,* there is a region referred to as a pathogenicity island containing gene sequences for the expression of proteins associated with virulence (vap, "virulence-associated protein") (TAKAI, S., HINES, S. A., SEKIZAKI, T., NICHOLSON, V. M., ALPERIN, D. A., OSAKI, M., TAKAMATSU, D., NAKAMURA, M., SUZUKI, K., OGINO, N., KAKUDA, T., DAN, H., PRESCOTT, J. F. DNA sequence and comparison of virulence plasmids from Rhodococcus equi ATCC 33701 and 103. Infection and Immunity, 2000 Dec;68(12):6840-7). These Vap genes have been classified under various subtypes and are distributed in the pathogenicity island in a triple grouping, VapA, VapC and VapD, in the duo VapE and VapF, or in a different individual distribution, as observed for the remaining VapG and VapH. These plasmids can be present in multiple copies in the same bacterial cell and are frequently lost when they are cultivated repeatedly in nutrient broth at 38°C (TAKAI, S., KOIKE, K., OHBUSHI, S., IZUMI, C., TSUBAKI, S. Identification of 15- to 17-kilodalton antigens associated with virulent Rhodococcus equi. Journal of Clinical Microbiology. 1991 Mar;29(3):439-43). Despite having other elements associated with virulence, such as a polysaccharide capsule and production of lipolytic enzymes, the plasmids of *R. equi* are considered essential for pathogenesis (GIGUÈRE, S., HONDALUS, M. K., YAGER, J. A., DARRAH, P., MOSSER, D. M., PRESCOTT, J. F. Role of the 85-kilobase plasmid and plasmid-encoded virulence-associated protein A in intracellular survival and virulence of Rhodococcus equi. Infection and Immunity, 1999 Jul;67(7):3548-57). In this context, the strains that are characterized by the production of subtype A (VapA), produced from the vapA gene, have been described as potential pathogens for foals (TAN, C., PRESCOTT, J. F., PATTERSON, M. C., NICHOLSON, V. M. Molecular characterization of a lipid-modified virulence-associated protein of Rhodococcus equi and its potential in protective immunity. Canadian Journal of Veterinary Research. 1995 Jan;59(1):51-9; VON BARGEN, K., HAAS, A. Molecular and infection biology of the horse pathogen Rhodococcus equi. FEMS Microbiology Reviews. 2009 Sep;33(5):870-91. Epub 2009 Apr 23).

The functions of VapA and of other subtypes of Vap are still unknown. It is known that VapA is a lipoprotein, which remains firmly bound to the surface of the bacterial cell. Besides VapA, the subtypes C, D, E, G and H also exhibit similar functional characteristics, containing signaling peptide sequences for secretion or location on the cell surface and are also related to virulent strains of *R. equi* (KOHLER, A. K., STONE, D. M., HINES, M. T., BYRNE, B. A., ALPERIN, D. C., NORTON, L. K., HINES, S. A. Rhodococcus equi secreted antigens are immunogenic and stimulate a type 1 recall response in the lungs of horses immune to R. equi infection. Infection and Immunity. 2003 Nov;71(11):6329-37).

As secretion products or surface molecules, it is very likely that these subtypes exhibit characteristics of interaction with host molecules, enabling the survival thereof on the interior of cells of the immune system. These proteins exhibit a certain degree of identicalness among themselves in the amino acid composition, which vary from 39 to 76%, and the degree of homology is greater as the carboxy-terminal end of their molecules (BYRNE, B. A., PRESCOTT, J. F., PALMER, G. H., TAKAI, S., NICHOLSON, V. M., ALPERIN, D. C., HINES, S. A. Virulence plasmid of Rhodococcus equi contains inducible gene family encoding secreted proteins. Infection and Immunity. 2001 Feb;69(2):650-6).

The primary targets of infections by *R. equi* are foals up to four months old and having severe pneumonia, culminating in a clinical picture of pulmonary abscesses. Without treatment with antibiotics, mortality is up to 80%, but is reduced to about 12% with the establishment of antibacterial chemotherapy using a combination of rifampicin and erythromycin (VON BARGEN, K. HAAS, A. Molecular and infection biology of the horse pathogen Rhodococcus equi. FEMS Microbiology Reviews. 2009 Sep;33(5):870-91. Epub 2009 Apr 23).

These microorganisms are extremely resistant under a very broad range of environmental conditions and find in animal manure precisely the desirable characteristics for reproduction. In this environment, young animals with their still-developing immune system can inhale powdery dispersions containing viable bacterial cells, which is the probably mechanism of transmission of pneumonia due to *R. equi.* Once inhaled, *R. equi* is absorbed by defense cells of the pulmonary epithelium (alveolar macrophages) and reside on the interior of these cells, and they have mechanisms to escape death on the interior of cytoplasmic vesicles (VON BARGEN, K., HAAS, A. Molecular and infection biology of the horse pathogen Rhodococcus equi. FEMS Microbiology Reviews. 2009 Sep;33(5):870-91. Epub 2009 Apr 23). The pulmonary lesions occur as a result of the recruitment of defense cells to the area of infection. Neutrophils and macrophages thus fill the alveolar space, creating an inflammatory environment that progresses to necrotic lesions of the pulmonary parenchyma. Granulomatous lesions are common and can break, dispersing *R. equi* to other organs of the host.

The high level of bacterial discharge presented in infected animals leads to self-ingestion of large quantities of bacterial cells, which may result in diarrhea with a clinical picture of ulcerative enteritis (CIMPRICH, R. E., ROONEY, J. R. Corynebacterium equi enteritis in foals. Veterinary Pathology. 1977 Mar;14(2):95-102). This entire context illustrates a probable cycle of *R. equi,* which is eliminated in the environment by infected animals and finds a new host in the vicinity.

Foals of up to six months of age are more susceptible to infection, with greater incidence in animals from birth to two months, and the disease occurs primarily in environments in which there is horse breeding. The infectious status is variable in duration, generally around three weeks, and is associated with a high mortality rate. The first signs of progressive chronic disease are fever above 41 °C, increased respiratory rate, bronchovesicular wheezing with wheezing in the airways, coughing, runny nose and depression. A clinical picture of severe diarrhea is common and indicates the colonization of the intestinal mucosa by *R. equi.* The symptoms can progress to a state of respiratory failure, and an untreated animal succumbs to asphyxiation and dies. Occasionally, the disease can progress to an acute form, killing the animal in less than 48 hours.

The treatment of infections by *R. equi* is lengthy and consists of the combination of two or more antibiotics. Appropriate treatment, adopted from the initial stages of infection by sensitive strains, must result in a cure. However, the primary drawback of prolonged treatment with antibiotics is its elevated cost, besides favoring the emergence of resistant strains. Using infection in foals as a point of reference, the most optimistic scenario is provided by the cure, with the usual result of the reduction of the athletic ability of the affected animals, which has a negative impact on the horse trade. All of these factors point out the need for the development of alternative strategies for preventing infections by *R. equi.*

Considering the role of VapA in the virulence of *R. equi,* it is reasonable that the development of vaccines be based on that subtype. However, efforts to construct vaccines for the production of this constituent using recombinant DNA technology have not yielded satisfactory results (VANNIASINKAM, T., BARTON, M. D. HEUZENROEDER, M. W. Immune response to vaccines based upon the VapA protein of the horse pathogen, Rhodococcus equi, in a murine model. International Journal of Medical Microbiology. 2005 Jan;294(7):437-45), although we find some promising results (OLIVEIRA, A. F., FERRAZ, L. C., BROCCHI, M., ROQUE-BARREIRA, M. C. Oral administration of a live attenuated Salmonella vaccine strain expressing the VapA protein induces protection against infection by Rhodococcus equi. Microbes and Infection. 2007 Mar;9(3):382-90. Epub 2007 Jan 12).

Various attempts at attenuating *R. equi* for use in vaccination have not been successful. Up to the present time, the only vaccine available for the prevention of equine *Rhodococcus* is composed of a lipoprotein extract, enriched with VapA, designed for subcutaneous application in pregnant mares for the passive immunization of the foals. The efficacy of this vaccine has yet to be demonstrated (http://www.labproser.com.ar/prodce_01en.htm).

For the elimination of *R. equi,* as with other intracellular pathogens, it is important that there be modulation for a cell-mediated immune response (Th1), since antibody-mediated responses (Th2) are inefficient at neutralizing these pathogens. Results of experimental models have shown that the desirable aspect in vaccine preparations must be the induction of high levels of immunoglobulin G isotope 2a (IgG2a), the secretion of which is stimulated by interferon gamma, a typical cytokine of responses of type Th1. In this context, the construction of a vaccine based on the VapA antigen, which induces a Th1 pattern response would be the exact model for the success of a vaccine preparation. However, VapA-based vaccine constructions, which classically modulate the immune response into a Th1 pattern, have not been able to protect animals against infection by *R*. *equi* (VON BARGEN, K., HAAS, A. Molecular and infection biology of the horse pathogen Rhodococcus equi. FEMS Microbiology Reviews. 2009 Sep;33(5):870-91. Epub 2009 Apr 23).

Various questions about the biology of infection by *R. equi* have yet to be answered. There are several accounts regarding the association between the virulent capacity of strains and the expression of the VapA antigen. However, the genome of *R*. *equi* has been described as containing a series of cell-surface proteins that may be involved in the interaction with cells of the host during the infectious process (VAZQUEZ-BOLAND, J. A., PRESCOTT, J. F., MEIJER, W. G., LEADON, D. P., HINES, S. A. Rhodococcus equi comes of age. Equine Veterinary Journal. 2009 Jan;41(1):93-5).

International publication WO 1988/009669 relates to vaccines for immunization comprising pathogenic microorganisms or avirulent derivatives that are not capable of producing functional cyclase adenylate and cyclic AMP receptor protein.

Brazilian patent application PI 0414353 provides pyroplasmid proteins (*Boophilus microplus*)*,* nucleic acid, cDNA fragment, recombinant DNA molecule, live recombinant vehicle, host cell, vaccine, method for the preparation of a vaccine, uses of a protein, of a nucleic acid sequence and diagnostic test.

European patent EP 827410 shows a method for introducing endogenous or foreign genes into animal cells using live bacteria as vectors.

European patent EP 500699 describes methods for individual immunization for protection against infections by Gram-negative bacteria, vaccine compositions containing live, avirulent *Salmonella.*

European patent EP 1537214 presents a vector-host system exhibiting a regulation of the lysis of bacteria to enable the release of genetic vaccine vectors or desirable gene products into a eukaryotic cell.

It should be emphasized that the expression of the VapG antigen by *R. equi* is high, both in the pulmonary tissue and in *in vitro* culture, and it is greater than all of the other Vap antigens (JACKS, S., GIGUÈRE, S., PRESCOTT, J. F. In vivo expression of and cell-mediated immune responses to the plasmid-encoded virulence-associated proteins of Rhodococcus equi in foals. Clinical Vaccine Immunology. 2007 Apr; 14(4):369-74. Epub 2007 Feb 14). Nevertheless, this constituent has not yet been used in vaccine strategies up to the present time, which indicates that the use of this factor as an immunogen may have been underestimated.

Thus, unexpectedly, the Applicant developed vaccine compositions containing vectors that produce an immune response against *R*. *equi.*

### DESCRIPTION OF THE FIGURES

Figure 1 presents [*sic*] the electrophoretic profile in agarose gel of polynucleotide amplified by polymerase chain reaction (PCR), using the DNA of the recombinant vaccine strains of *Salmonella* chi-3987 as a mold.
Figure 2 shows a diagram of a recombinant *asd*+ plasmid, showing the location of the VapG gene.
Figure 3 shows the results for the persistence of recombinant strains, control (pYA control) and only expressing VapG (vapG), in the spleen (3a) and in the liver (3b) of mice. The persistence is measured by counting the bacterial colony-forming units (CFUs) in the organs of the animals.
Figure 4 presents the results for the recovery of *R. equi* cells in the spleen and liver of mice, immunized with vaccine strains of *Salmonella* and challenged with an intravenous dose of one hundred million *R. equi* cells.

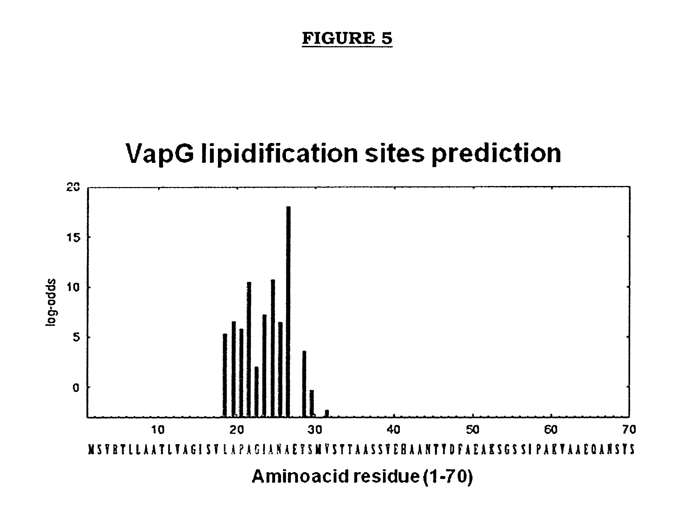

Figure 5 shows the first 70 amino acids of the polypeptide sequence of VapG and highlights, in gray, the residues involved at the cleavage site of that lipoprotein. The bars indicate probable cleavage sites of the signal peptide, and the heavier bar indicates greater probability of cleavage between the alaninyl residues ("A") and glutamyl residues ("E").
Figure 6 shows the electrophoretic profile (SDS-PAGE) of the proteins extracted from cells of attenuated *Salmonella,* fully expressing the recombinant VapG antigen of *R*. *equi,* pointed out by a black circle.
Figure 7 shows the interferon gamma pattern produced as a characteristic of Th1 immune response to *R. equi,* through immunization with the vaccine strain of *Salmonella* (vapG). *p<0.05.

### DESCRIPTION OF THE INVENTION

The present invention provides live recombinant microorganisms such as prokaryote organisms, particularly enterobacteria, preferably *Salmonella enterica,* containing SEQ. ID. no. 1 and SEQ. ID. no. 2 capable of expressing VapG and/or VapA/VapG lipoproteins (SEQ. ID. no. 3 and SEQ. ID. no. 4), optionally in combination with other active ingredients, methods for preparing vaccine strains, antigens (SEQ. ID. no. 3 and SEQ. ID. no. 4) and vaccine compositions, preferably vectored vaccines. Additionally, the present patent application relates to the use of the vaccine vectors in the preparation of pharmaceutical compositions, particularly vaccines indicated for the prevention and/or treatment of infections by *Rhodococcus equi,* its antibodies and/or antisera, diagnostic kits and methods for the prophylaxis and/or treatment of infections by *R. equi.*

In general, the microorganisms of the present application comprise live, non-pathogenic and recombinant microbiological vectors capable of expressing at least one polypeptide of *R. equi* and presenting to the immune system in the surroundings of a receptor in order to function as an antigen presenter of heterologous origin and generate an immune response to *R. equi.*

In a first embodiment, the present invention is addressed to live recombinant microorganisms such as prokaryotic organisms, particularly enterobacteria, preferably *Salmonella enterica,* containing SEQ. ID. no. 1 and SEQ. ID. no. 2 capable of expressing VapG and/or VapA/VapG lipoproteins (SEQ. ID. no. 3 and SEQ. ID. no. 4) and/or derivatives thereof, as vaccine vectors.

Particularly, the heterologous nucleic acid (SEQ. ID. no. 1) codifies for the VapG antigen of *R. equi* (SEQ. ID. no. 3) and the heterologous nucleic acid (SEQ. ID. no. 4). The polypeptide *of R. equi* can, however, contain other derivations of the VapG antigen such as, for example, VapA, VapC, VapD, VapE, VapF, VapH and/or homologous polypeptides, where portions of the N-terminal region are eliminated, suppressing the peptide signal and the lipidification sites of the molecule.

The N-terminal or C-terminal portions represent the starting and ending portions of the translation of a protein or polypeptide, respectively. For the polypeptide chains of the present application (SEQ. ID. no. 3 and SEQ. ID. no. 4), the translations are initiated in the methionine residue numbered as "1" ("M").

The polypeptides of the present invention may be from 55% to 100% identical, preferably 70% identical to the VapG protein of *R. equi,* including any resulting variations and fragments, which are capable of stimulating an immune response to R. *equi.* In particular, the immunogenic polypeptide comprises the contiguous sequence starting from at least 15 amino acids from any characteristic portions of the VapG protein of *R. equi,* preferably with 10 identical amino acids in the portions of the VapG protein of *R. equi,* being from 55% to 100% identical, preferably 70% identical to the mold portion of the VapG protein *of R. equi* (SEQ. ID. no. 3), and still more preferably, comprising an amino acid sequence from 1 to 161 or 11 to 172 of VapG (SEQ. ID. no. 3) or its related fragments. Nevertheless, the VapG protein (SEQ. ID. no. 3) presents extracted N-terminal portions without the first 5 to 50 amino acids of the N-terminal portion or without the last 5 to 50 amino acids of the C-terminal portion. The polypeptides of *R. equi* can be expressed in the form of fusion proteins or separately in the form of multiple polypeptides. Optionally, the polypeptide of *R. equi* can be expressed in the form of a fusion protein comprising polypeptide sequences of the VapG and/or VapA antigens. Optionally, the polypeptide sequences can be associated with other active components such as, for example, other Vap proteins and/or adjuvants for modulating the immune response.

The polynucleotides described in the present application are preferably contained in an extrachromosomal vector (plasmid); however, they can be integrated in the bacterial chromosome.

In a second embodiment, this invention describes the method for the preparation of vaccine strains of *Salmonella* comprising: i) mutations for attenuating the virulence; ii) one or more genetic expression fragments, with each fragment comprising a sequence of amino acids of heterologous origin (SEQ. ID. no. 1 and SEQ. ID. no. 2) under the transcriptional control of a constitutive or inducible promoter where the amino acid sequence is oriented so as to codify, partially and/or completely, a virulence antigen of *R. equi* (SEQ. ID. no.3 and SEQ. ID. no. 4) and, optionally, iii) a mutation in a gene essential for its reproduction, rendering it auxotrophic for components that are added in the culture medium without the need for the use of antibiotics for the selection.

Particularly, the attenuated strains of *Salmonella* used in the present invention comprise the strains which contain mutations in genes responsible for the synthesis of aromatic compounds (delta-*aro*), in genes that interfere with physiological mechanisms of the host (delta-*cya* and delta-*crp*), or in genes that codify for the expression of nucleic acid-binding bacterial proteins (delta-*fis*, delta-*dps*, delta-*hfq*, delta-*hup*A, delta-*hup*B or delta-*hha*). Preferably, the attenuated strains of *Salmonella* contain a mutation in the *aro* gene (delta-*aro*) or an attenuated strain of *Salmonella* contains mutations in the *cya* and *crp* genes (delta-*cya* and delta-*crp*).

Another mutation strategy for the selection of recombinant strains of *Salmonella* and guaranteeing the stability of a plasmid constructed for the expression of the recombinant protein involves the mutation of a gene associated with the biosynthesis of components of the cell wall, such as mutation in the *asd* gene, which codifies for aspartate-semialdehyde dehydrogenase, an enzyme that participates in the synthesis of structures of the bacterial cell wall and renders the bacterial cell dependent on diaminopimelic acid for growth. In this configuration, the *asd* gene can be supplemented through the introduction of a non-plasmid gene fragment with all of the characteristics for the expression of the *asd* gene. Therefore, the result of the introduction of these plasmids into delta-*asd* bacterial strains, where delta refers to the absence or deactivation of the gene, is the selection of recombinant clones in the culture medium without supplementation, and that they express the recombinant protein of *R. equi.* Finally, the attenuating mutation is the result of the absence or deactivation of at least one gene associated with its pathogenicity, the attenuated *Salmonella* being derived from the parental strain *Salmonella enterica* serotype Typhimurium UK-1.

The constitutive promoter guides the expression of the recombinant protein independently of the growth phase of the bacterial cell, of the cell metabolism or the presence of inductors. Also, the inducible promoter orients the expression of the recombinant protein under conditions of nutritional and/or oxidative stress or through the addition of an inductive chemical component. As an optional example, an inducible promoter can be used *in vivo* depending on the structures of the recipient organism and guiding the expression of the recombinant protein through the process of the transcription of the cells of the host.

The attenuated strain of *Salmonella enterica* used in the present application comprises a mutation in the *aroA* gene which codifies for the enzyme 5-enol-piruvl-shikimato-3-phosphate synthase (EPSPS), which is a common precursor in the biosynthesis of numerous aromatic compounds in bacteria and other organisms. Particularly, the delta-*aro* strain used is H683 of *S*. *enterica* serovar Typhimurium.

The present patent application also discloses the use of promoters for expression in bacteria, such as the tac, pac, rac, trc, lpp, dps or recA promoters from enterobacteria, T3, T7 and/or SP6 promoters from bacteriophages. Also, in the case of expression in eukaryotic cells, MET2, MET25 promoters from yeasts, RSV, CMV, HTLV and SV40 promoters from viruses and beta-Actin, mPGK, eiF4alpha, CAG and ENS-I promoters from mammals.

In one particular configuration, the polypeptides of *R. equi* can contain codon adjustments for expression in different vectors, preferably *Escherichia coli,* for example, the strain delta-*asd* chi-1776.

In a third embodiment, the present invention relates to the VapG antigen of *R*. *equi* (SEQ. ID. no. 3) expressed so as not to contain the lipidification sites where, optionally, it can include the VapG antigen in combination with other Vap antigens of *R*. *equi* selected from a group of polypeptides lacking up to 50 amino acids from the N-terminal portion (SEQ. ID. no. 3 and SEQ. ID. no. 4).

The recombinant antigen (SEQ. ID. no. 3 and SEQ. ID. no. 4) can be produced and purified by electrophoresis, filtration, chromatograph, centrifugation, and/or combinations of these methods. Moreover, the substantially purified antigen can contain from 60 to 99% of the substance in isolation, since it does not contain toxic byproducts of purification.

In a fourth embodiment, the present invention relates to vaccine compositions containing the recombinant vaccine vector (SEQ. ID. no. 1 and SEQ. ID. no. 2) capable of expressing VapG and/or VapA/VapG lipoproteins (SEQ. ID. no. 3 and SEQ. ID. no. 4) in pharmaceutically suitable vehicles, preferably vectored vaccines.

The vaccine composition may also comprise a population of homogeneous bacterial cells. However, the compositions may also contain a plurality of bacterial cells from different isolates such as, for example, a homogeneous population of attenuated *Salmonella* which comprises a population of cells capable of expressing the VapG antigen *of R. equi* and a plurality of cells which comprises more than one population of attenuated *Salmonella,* with one population of cells expressing VapG of *R. equi* and a second population expressing VapA, VapC and/or VapH.

The vaccine composition of the present invention can contain a combination of a purified recombinant antigen such as a population of attenuated *Salmonella* capable of expressing VapG in combination with the purified recombinant VapA, VapC and/or VapH antigens in a pharmaceutically suitable vehicle and/or diluent. In another configuration, the vaccine composition contains attenuated *Salmonella* capable of expressing fusions of recombinant antigens comprising two or more immunogenic sequences of antigens of *R. equi* fused in a single structure. The recombinant antigens can be fused by other peptide sequences and/or immunostimulatory molecules such as cytokines and chemokines.

The present invention also encompasses the co-expression of one or more genes that codify for immunostimulatory proteins, like cytokines and chemokines such as IFN-alpha, IFN-beta, IFN-gamma, IL-2, IL-3, IL-4, IL-5, IL-6, IL-7, IL-8, IL-9, 1L-10, IL-11, IL-12, IL-13, IL- 14, IL-15 or IL-16. In this case, co-expression is defined as the construction of vectors which comprise the polynucleotide fragment of heterologous origin for the expression of one of more cytokine and/or chemokine genes, in combination with another polynucleotide fragment of heterologous origin for the expression of polypeptide(s) related to VapG of *R*. *equi.*

The compositions of the present patent application can also contain adjuvants such as, for example, a CpG oligodeoxynucleotide adjuvant, comprised of aluminum (aluminum hydroxide, aluminum oxide or aluminum phosphate), oily adjuvants (mineral oil, complete or incomplete Freund's adjuvants), adjuvants originating from mycolic acid (trehalose dimycolate), bacterial liposaccharides (LPS), peptidoglycans (mucopeptide), glycoproteins (N-Opaca, muramyl dipeptide analogs), proteoglycans, streptoccocal preparations (OK432), saponin, neutral oils (miglyol), vegetable oils, liposomes, polyols, vitamin E, Carbopol, interleukins and/or combinations thereof.

In addition, the compositions of the invention can contain pharmaceutically suitable excipients and/or carriers, like liquid solutions such as saline, hypochlorite and/or other non-toxic salts with near-physiological osmolarities, a solid solvent or a material for encapsulation where the vaccine can be resuspended or dissolved. The pharmaceutically suitable carrier must also be non-toxic to the recipient organism and compatible with the attenuated live microorganism. The pharmaceutical excipients and vehicles can be selected from among amide, glucose, lactose, sucrose, gelatin, malt, rice, flower, chalk, silica gel, sodium stearate, magnesium stearate, glycerol monostearate, ascorbic acid, talc, sodium chloride, powdered milk, maltodextrin, glycerol, propylene, glycol, water, ethanol, preservatives, sweeteners and/or combinations thereof.

Particularly, one configuration of the invention provides a vaccine composition which comprises a lyophilized microorganism, particularly an attenuated strain of *S. enterica* serovar Typhimurium, with deletions in the *cya, crp* and *asd* genes (delta*-cya,* delta-*crp* and delta-*asd*) in suspension containing at least one excipient selected from among lactose, sorbitol, dibasic sodium phosphate, monobasic potassium phosphate, sucrose, sterile saline and/or water.

The compositions of the present invention can be administered by invasive methods such as, for example, parenterally, subcutaneously, intravenously, intramuscularly, intraperitoneally, transdermally, intramucously, intranasally, intragastrically and/or through non-invasive methods such as orally, rectally, by inhalation, nasally and/or ocularly, preferably orally or nasally.

The pharmaceutical forms of the present patent application can vary between gelatin capsules, pills, tablets, dragees, elixirs, suspensions, syrups, suspensions for vaporizers, preferably vaccines. Optionally, gelatin capsules can be used as carriers for lyophilized vaccines.

In a fifth embodiment, the present invention is addressed to the use of vectors in the preparation of pharmaceutical preparations, particularly vectored vaccines indicated for the prevention and/or treatment of infections by *Rhodococcus equi.*

In particular, the infections refer to conditions associated with infection by *R*. *equi,* such as pyogranulatomous pneumonia and/or colitis.

The immune response is characterized by a pattern of cell-mediated immunity associated with a mucous immune response, with production of immunoglobulin isotype A (IgA), specifically for the recombinant protein of *R. equi.*

In a sixth embodiment, the present invention includes antisera, antibodies, in isolation or substantially purified, produced against the recombinant antigens (SEQ. ID. no. 3 and SEQ. ID. no. 4).

The antisera and antibodies can be purified by means of affinity chromatography with the recombinant antigens.

In a seventh embodiment, the invention relates to a diagnostic kit containing one or more recombinant Vap antigens (SEQ. ID. no. 3 and SEQ. ID. no. 4) of *R. equi* (SEQ. ID. no. I and SEQ. ID. no. 2) in conjunction with its specific antibodies. In addition, the kit is designed for immunoenzymatic assay (ELISA) for the diagnosis of infections by *R*. *equi.*

In an eighth embodiment, the present invention presents methods for prophylaxis and/or treatment based on two immunizations, one being the initial immunization and the other being a booster, suitable for the administration of vaccines. Optionally, in the two-immunization method, at least one vaccine composition of the present invention is used, it being possible to administer one or more vaccine compositions to a recipient organism presenting the recombinant polypeptide in the context of the vaccine vector for the immune system of the recipient, which must be a vertebrate, and a second composition being used as a booster dose.

In an immunization protocol of the present invention, the attenuated recombinant strain of *Salmonella* is used to present antigen(s) of *R. equi* to the immune system and administered in a certain composition during the first day of life of a vertebrate animal, a second dose with a composition identical to that of the first being used as a booster for an immune response to *R. equi,* administered fifteen days after the first dose.

Particularly, the method for vaccinating a recipient organism against *R. equi* is performed by oral administration for a horse with a high risk of contracting an infection by *R. equi* or a horse that is already infected by *R. equi;* this includes other vertebrate hosts as well.

With the purpose of better defining the present invention, following is a list of some definitions for the terminology used:
The term "attenuated" refers to a microorganism genetically modified for the purpose of not causing illnesses in humans or human models.
"Vaccine vector" refers to an avirulent bacteria, virus or circular nucleic acid molecule used for the expression of heterologous antigens in the environment of a host and constructed so as to stimulate a protective immune response, assembled from the recombinant antigen as part of the microorganism that produces it naturally.

The term "immune response" refers to the mounting of a response by the host which confers protective immunity and can be regarded as a sufficient response to prevent the recipient organism from developing symptoms related to the infection.

The term "gene expression fragment" refers to a construction of nucleic acids comprising a nucleic acid of heterologous origin that is under the transcriptional control of a promoter.

The term "immunogenic polypeptide" refers to a portion of a protein associated with the virulence of *R. equi* or to the entire protein as it is expressed naturally by *R*. *equi.* The immunogenic polypeptide must be capable of stimulating an immune response when expressed by the bacterial vaccine vector in the vicinity of the recipient organism.

The term "pharmaceutically suitable" refers to a composition that has been approved by regulatory agencies or included in the National and/or Internationally recognized Pharmacopoeias.

The term "promoter" refers to a sequence of nucleic acid nucleotides responsible for the linking of complex proteins which comprise an RNA polymerase enzyme, resulting in the initiation of the transcription of a juxtaposed gene.

The terms "nucleic acid," "nucleotide" and "polynucleotide" used in the descriptions refer to deoxyribonucleotides (DNA) or ribonucleotides and polymers thereof, whether in the form of a single or a double strand. These terms are related to nucleic acids that contain natural nucleotide analogs, which have identical binding properties to those of the nucleic acids of reference and are metabolized in a similar manner as nucleotides found naturally. The term "nucleic acid" is also used for describing genes, complementary DNA (cDNA), and the messenger RNA (mRNA) transcribed in a gene. Also with regard to the terms "nucleic acid," "nucleotide" and "polynucleotide," they comprise DNA molecules of any origin (cDNA or genomic), RNA molecules, [and] DNA or RNA analog molecules created from reactions with analog nucleotides.

The term "identity," in relation to sequences of nucleic acids and proteins, refers to the comparison of two or more polynucleotide sequences or of two or more polypeptide sequences, respectively.

The term "transformation" is used to refer to the transfer of nucleic acid to the inside of a cell, and the term "genetic transformation" refers to the transfer of nucleic acid to the transfer and incorporation of recombinant DNA on the inside of a cell.

The term "variant" is used to refer to polynucleotides or polypeptides that exhibit differences when compared to the nucleic acid or polypeptides of reference but maintain their essential properties.

The term "similar" refers to the fact that the exchange of an amino acid from a particular position in a polypeptide chain does not result in functional or structural alteration when compared to the molecule of reference.

### EXAMPLES

The strains and plasmids were obtained from commercial sources and were used in accordance with the manufacturers. The identity and purity of all of the compounds were verified by means LC-MS and RNH HI analysis.

### CONSTRUCTION OF THE PLASMID FOR THE EXPRESSION OF VAPG OF R. EQUI IN ATTENUATED STRAINS OF SALMONELLA

The bacterial strains used in the present invention are described in table 1. The strain of *Salmonella* used was delta-*cya*, *-crp* and *-asd,* called chi-3987.

**TABLE 1**

| **BACTERIAL STRAINS** | |
|---|---|
| **Bacteria** | **Genotype** |
| *E. coli* chi.6212 | F*'endA*1 *hsd*R17 (rₖ*-*mₖ+) *sup*E44 *thi*-1 reaA1 *gyrA* (Nalr) *rel*A1 (Δ(laclZYA-argF) U169 *deo*R (ϕ80 *diac*Δ(lacZ) M15) Δasd |
| *S. enterica* Typhimurium chi.3987 UK-1 | pStV1+ Δ*cya*-12 Δ*crp-*11 Δ*asd*A1 Δ[zhf-4::Tn10] |
| *S. enterica* Typhimurium H683 | Δ*aro*Δ*asd* |

The structural sequence of the *vap*G gene was obtained through DNA amplification reaction (PCR) using the initiators (SEQ. ID. no. 5 and SEQ. ID. no. 6) containing cleavage sites for the endonuclease enzymes *Bam*H1 e *Sal*I.

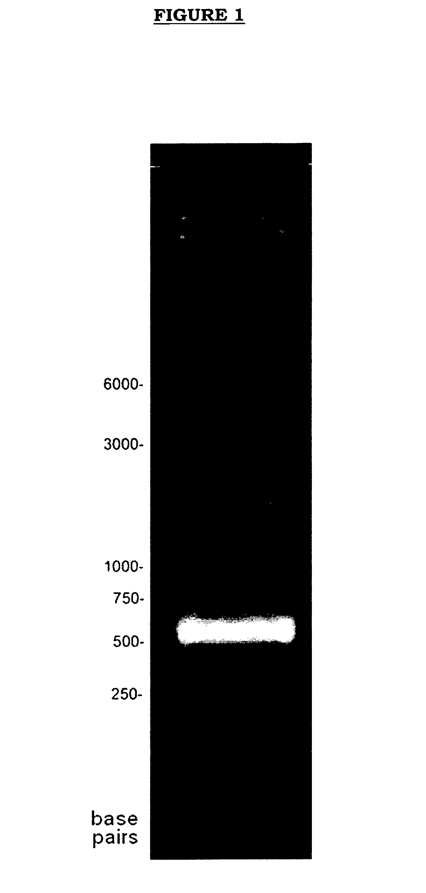

The amplification by PCR was performed on a final volume of 50 microliters using a collection of commonly used reagents, and the product of that reaction (SEQ. ID. no. 1) is shown in figure 1. The amplification reaction was performed using methods described in the prior art. With the amplification, the fragments obtained were constituted from the cleavage sites by the *Bam*HI and *Sal*I endonucleases, flanking the structural gene *vap*G in order to thus enable the cloning of the amplified fragment, digested by these endonucleases, in the *asd*+ plasmid, digested with the same endonucleases.

The digestion and purification procedures were performed using available reagents. The linking of the amplification fragment to the asd+ plasmid, both digested and purified, was performed through a reaction with DNA ligase T4 enzyme.

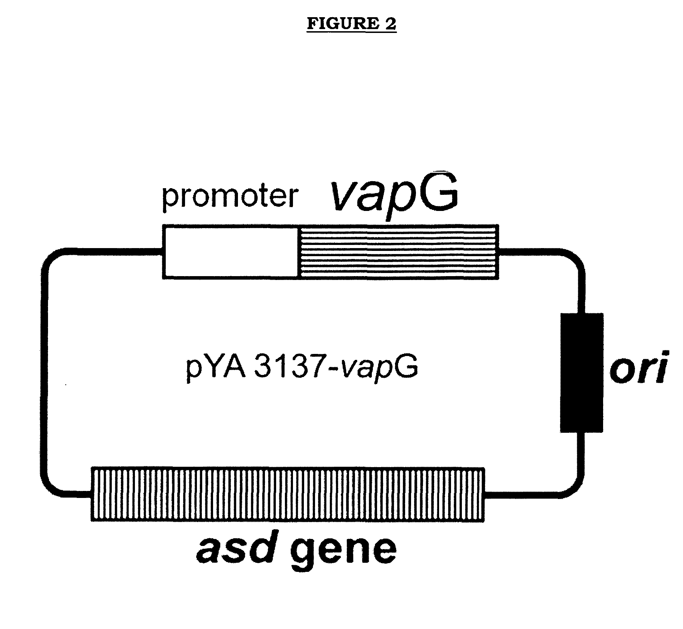

The expression cassette of the VapG antigen was inserted into *asd*+ plasmid through the restriction sites of the *Bam*HI and *Sal*I endonucleases. This construction was made without codon optimization, but the polynucleotides that codify VapG can contain modifications that do not alter the polypeptide expression sequence. After the modification of the *asd*+ plasmid with the introduction of the expression cassette of vapG, this recombinant plasmid is ready for introduction by electroporation into cloning strains, such as *Escherichia coli* delta-*asd* chi.6212, and into vaccine strains of *Salmonella.* The recombinant *asd*+ plasmid (*vap*G) has been schematized in figure 2.

The electroporation process was performed through the addition of 5.0 microliters of a suspension of recombinant plasmid to 40 microliters of electrocompetent bacterial cells. The electroporation was performed in a BioRad electroporator under conditions of 12.5 kilovolts/cm, 200 ohms and 25 microFaraday. The electroporation processes were first performed on cloning cells (*E. coli* chi.6212) for subsequent extraction of plasmids and introduction into *Salmonella* vaccine cells (chi.3987). All of the clones were selected based on growth in artificial solid culture media without the addition of supplements. Delta-*asd* bacterial clones carrying the *asd*+ plasmid grew in these culture media without the addition of diaminopimelic acid supplement, indicating the activity of these plasmids for the selected clones. Some smaller clones were able to grow after incubation for one day and encircle larger clones, these being satellite colonies that take advantage of the metabolism of the nearby positive clones in order to grow without carrying the recombinant *asd*+ plasmids.

Control strains were transformed with the *asd*+ plasmid empty, that is, without cloning cassettes. Selected colonies were cultivated in 3.0 milliliters of artificial nutrient broth. The bacterial suspensions in liquid medium were processed for extraction, analysis and sequencing of plasmid DNA, and a portion was separated for primary storage of samples. For the samples that were characterized as having the *vap*G gene, a subsequent culture was performed in enriched broth (Luria-Bertani, LB) to create cell banks for storage at negative temperature at 80°C. All of these procedures were the same for the vaccine clones and for the control clones containing only empty *asd*+ plasmid.

### IMMUNIZATION MODEL IN MICE

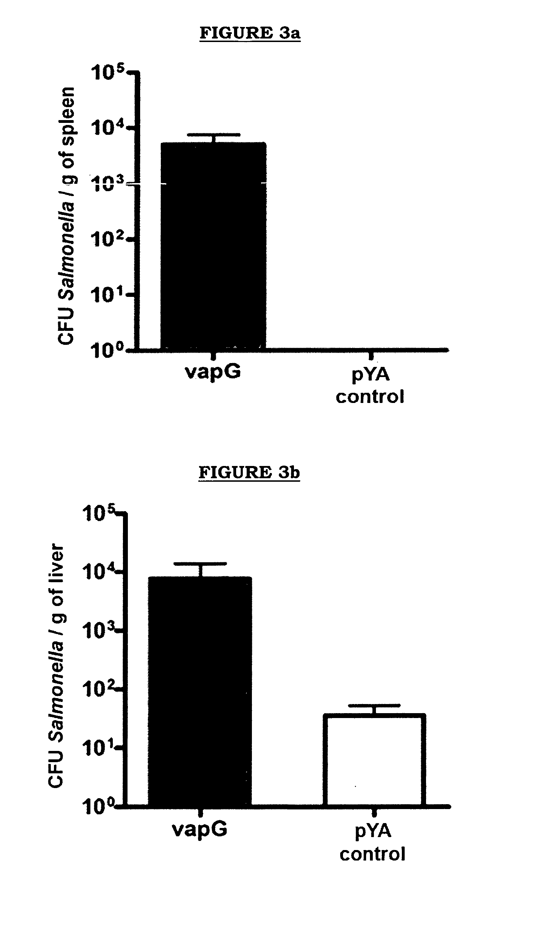

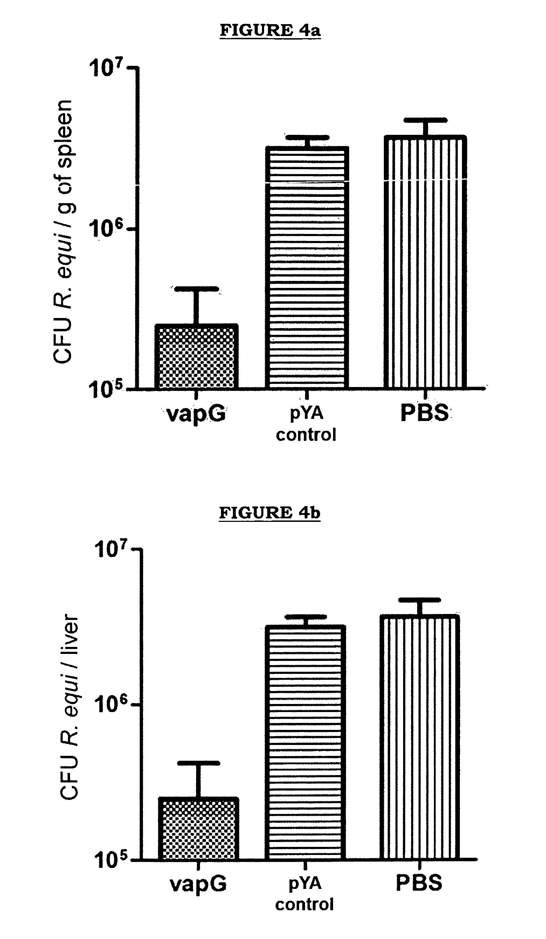

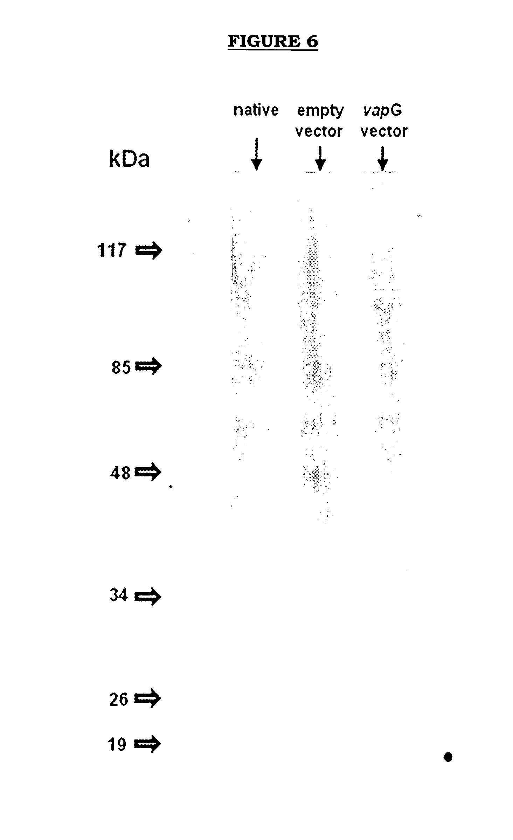

Isogenic female mice from the BALB/c line, between 6 and 8 weeks of age, were used. Two experimental groups were formed containing 12 BALB/c mice. In group I, the animals were immunized with the vaccine strain containing the recombinant *asd*+ plasmid for the *vap*G expression cassette, and in group II the animals were given the vaccine strain containing the empty *asd*+ plasmid. The mice were inoculated orally with 200 microliters of the bacterial suspensions in a single dose of approximately 100 million bacterial cells. Subgroups of 4 animals from each group were sacrificed on the third, tenth and twentieth days after inoculation, and the spleen and liver of these animals were removed to study the bacterial persistence in the tissues. These organs were first weighed and divulsed in phosphate buffer (PBS) using an electric homogenizer. 100 microliters of this homogeneous mixture was cultivated for one day in solid culture medium selective for Gram-negative bacteria (McConkey's agar). The quantification of bacterial cells was done by counting the colonies observed in the solid medium, and these were estimated in number of bacteria per gram of organ as shown in figure 3. To evaluate the induced protection, BALB/c mice were immunized according to the protocol described above, with a fourth group of animals that only received the suspension vehicle comprised of phosphate buffer (PBS). All of the animals were challenged with a lethal intravenous dose of about 100 million cells of R. equi of the strain ATCC33701. Five days after the challenge, the animals were euthanized in order to remove the spleen and liver and count the *R*. *equi* cells per gram of organ (figure 4) as described above. Colonies isolated from the tissues were evaluated with respect to the expression of Vap antigens through electrophoresis in polyacrimide gel as shown in figure 6.

For the oral immunization, two experimental groups of BALB/c mice were formed. In group I, the animals were immunized with a vaccine strain containing the recombinant *asd*+ plasmid for the expression cassette of *vap*G, and in group II the animals received the vaccine strain containing the empty *asd*+ plasmid. The mice were inoculated orally with 200 microliters of the bacterial suspensions containing about 100 million bacterial cells; two immunizations were performed with an interval of 14 days.

Blood was collected prior to the first immunization to obtain pre-immune (control) serum and after the second immunization in intervals of 7 days for a total of 6 weeks of immune (test) serum collection. At each sample point, serum was collected from four mice (n=28). Collection was performed subocularly and the serum was collected through centrifugation of the whole blood in order to determine the titer of antibodies (total IgG, IgGl and IgG2a).

The fecal material was collected in isolation (150-300 g/animal) and immediately transferred to microcentrifugation tubes containing 1.0 ml PBS, PMSF protease inhibitor 1.0 mM and bovine serum albumin (BSA) 1%. The tubes were incubated in a refrigerator for approximately 16 hours, and then the samples were homogenized by vortexing and centrifuged at high speed for 5 minutes. The supernatants were transferred to new tubes to determine the titer of the abovementioned immunoglobulins.

An immunoenzymatic assay (ELISA) was used to qualify, in the feces and in the serum of immunized mice, the presence of IgA, total IgG, IgGl and IgG2a (serum) antibodies that react with crude cell extract antigens of *Rhodococcus equi.* For these assays, 96-well polystyrene plates were sensitized with crude cell extract antigens of *R. equi* using as primary antibodies those contained in the serum or feces of the immunized animals. The reactions were detected using immunoenzymatic probes (anti-immunoglobulins linked to peroxidase enzyme), observing their respective classes and subclasses of immunoglobulins and exposed using commercially available substrate for peroxidase.

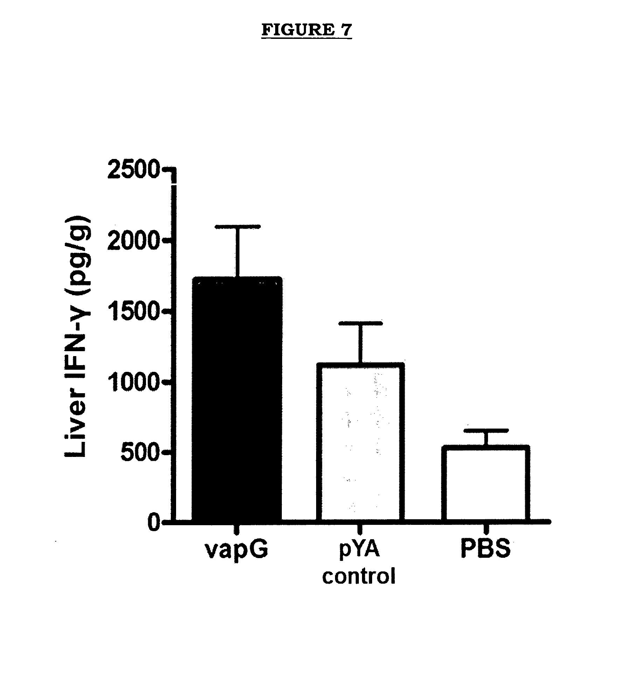

To evaluate the immune response pattern after immunization, four groups of BALB/c mice were subjected to the same immunization protocol as described above. Fifteen days after the first immunization, the animals were given a second dose of immunogen. 30 days after the first immunization, the animals were euthanized to remove the liver for interferon gamma assay using an immunoenzymatic method (ELISA) as shown in figure 7.

## Claims

1. RECOMBINANT LIVE MICROORGANISMS, **characterized in that** they comprise prokaryotic organisms, particularly enterobacteria, preferably *Salmonella enterica,* containing SEQ. ID. no. 1 and SEQ. ID. no. 2.

2. MICROORGANISMS as set forth in claim 1, **characterized in that** the microorganisms comprise the capability of expressing VapG and/or VapA/VapG lipoproteins (SEQ. ID. no. 3 and SEQ. ID. no. 4) and/or derivations thereof.

3. MICROORGANISMS as set forth in claim 2, **characterized in that** a heterologous nucleic acid (SEQ. ID. no. 1) comprises codification for the VapG antigen of *R. equi* (SEQ. ID. no. 3).

4. MICROORGANISMS as set forth in claim 2, **characterized in that** the heterologous nucleic acid (SEQ. ID. no. 2) comprises codification for the VapA/VapG antigen (SEQ. ID. no. 3 and SEQ. ID. no. 4).

5. MICROORGANISMS as set forth in claims 2 to 4, **characterized in that** the polypeptide of *R. equi* further comprises other derivations of the VapG antigen, such as VapA, VapC, VapD, VapE, VapF, VapH and/or homologous polypeptides.

6. MICROORGANISMS as set forth in claims 2 to 5, **characterized in that** the polypeptide comprises the elimination of portions of the N-terminal region with the signal peptide and the lipidification sites of the molecules suppressed.

7. MICROORGANISMS as set forth in claims 1 to 6, **characterized in that** the polypeptides are 55% to 100% identical, preferably 70% identical to the VapG protein of *R. equi,* including any resulting variations and fragments.

8. MICROORGANISMS as set forth in claims 1 to 7, **characterized in that** the immunogenic polypeptides comprise a sequence of at least 15 amino acids, preferably with 10 identical amino acids in the portions of the VapG protein of *R. equi.*

9. MICROORGANISMS as set forth in claim 8, **characterized in that** the polypeptides comprise, particularly, a sequence of amino acids of 1 to 161 or 11 to 172 of VapG (SEQ. ID. no. 3) or fragments thereof.

10. MICROORGANISMS as set forth in claims 1 to 9, **characterized in that** they comprise polypeptides of *R. equi* expressed in the form of fusion proteins or separately in the form of multiple polypeptides.

11. MICROORGANISMS as set forth in claims 1 to , **characterized in that** the polynucleotides comprise the integration/incorporation thereof into a bacterial extrachromosomal or non-chromosomal vector, preferably in a plasmid.

12. METHOD FOR PREPARING STRAINS
VACCINES as set forth in claims 1 to 11 **characterized in that** they comprise the steps: i) mutations for attenuating the virulence; ii) one or more genetic expression fragments, with each fragment comprising a sequence of amino acids of heterologous origin (SEQ. ID. no. 1 and SEQ. ID. no. 2) under the transcriptional control of a constitutive or inducible promoter where the amino acid sequence is oriented so as to codify, partially and/or completely, a virulence antigen *of R. equi* (SEQ. ID. no.3 and SEQ. ID. no. 4) and, optionally, iii) a mutation in a gene essential for its reproduction, rendering it auxotrophic for components that are added in the culture medium without the need for the use of antibiotics for the selection.

13. METHOD as set forth in claim 12, **characterized in that** the strains preferably comprise attenuated strains of *Salmonella.*

14. METHOD as set forth in claims 12 and 13, **characterized in that** the strains comprise mutations in genes responsible for the synthesis of aromatic compounds (delta-*aro*)*,* in genes that interfere with physiological host mechanisms (delta-*cya* and delta-*crp*) and/or in genes that codify for the expression of nucleic acid-binding bacterial proteins (delta-*fis*, delta-*dps*, delta-*hfq*, delta-*hitp*A, delta-*hup*B or delta-*hha*).

15. METHOD as set forth in claim 14, **characterized in that** the strains comprise the absence or deactivation of at least one gene associated with the pathogenicity thereof, the attenuated *Salmonella* being derived from the parental strain of *Salmonella enterica* serotype Typhimurium UK-1.

16. METHOD as set forth in claims 12 to 15, **characterized in that** the attenuated strains of *Salmonella* comprise a mutation in the roA gene, which codifies for the enzyme 5-enol-piruvl-shikimato-3-phosphate synthase (EPSPS), particularly strain H683 of *S. enterica* sorotype Typhimurium.

17. METHOD as set forth in claims 12 to 16, **characterized in that** is comprises the use of promoters for expression in bacteria, such as the promoters tac, pac, rac, trc, lpp, dps or recA of enterobacteria, T3, T7 and/or SP6 of bacteriophages.

18. METHOD as set forth in claim 17, **characterized in that** the method further comprises the use of promoters MET2, MET25 from yeasts, promoters RSV, CMV, HTLV and SV40 from viruses, and beta-Actin, mPGK, eiF4alpha, CAG and ENS-I from mammals in the case of expression in eukaryotic cells.

19. METHOD as set forth in claims 12 to 18, **characterized in that** is comprises codon adjustments for expression in different vectors.

20. ANTIGEN as set forth in claims I to 11, **characterized in that** it comprises SEQ. ID. no. 3 and SEQ. ID. no. 4.

21. ANTIGEN as set forth in claim 20, **characterized in that** it comprises its expression without the lipidification sites.

22. ANTIGEN as set forth in claims 20 and 21, **characterized in that** it optionally comprises the VapG antigen in combination with other Vap antigens of *R. equi* selected from a group of polypeptides exhibiting absences of up to 50 amino acids from the N-terminal portion.

23. ANTIGEN as set forth in claims 20 to 22, **characterized in that** the recombinant antigen comprises from 60 to 99% of the substance in isolation.

24. VACCINE COMPOSITIONS as set forth in claims 1 to 23, **characterized in that** they comprise vectored vaccines.

25. VACCINE COMPOSITIONS as set forth in claim 24, **characterized in that** that they comprise recombinant vaccine vectors (SEQ. ID. no. 1 and SEQ. ID. no. 2) capable of expressing VapG and/or VapA/VapG lipoproteins (SEQ. ID. no. 3 and SEQ. ID. no. 4) in pharmaceutically suitable vehicles.

26. VACCINE COMPOSITIONS as set forth in claims 24 and 25, **characterized in that** they comprise the combination of a purified recombinant antigen, such as a population of attenuated *Salmonella* capable of expressing VapG in combination with purified recombinant VapA, VapC and/or VapH antigens in a pharmaceutically suitable vehicle and/or diluent.

27. VACCINE COMPOSITIONS as set forth in claims 24 and 26, **characterized in that** they comprise, particularly, attenuated *Salmonella* capable of expressing fusions of recombinant antigens containing one or more immunogenic sequences of antigens of *R. equi* fused in a single structure.

28. VACCINE COMPOSITIONS as set forth in claims 24 and 27, **characterized in that** they optionally comprise fusion with other polypeptide sequences and/or immunostimulatory molecule such as cytokines and chemokines.

29. VACCINE COMPOSITIONS as set forth in claim 28, **characterized in that** the cytokines and chemokines comprise IFN-alpha, IFN-beta, IFN-gamma, IL-2, IL-3, IL-4, IL-5, IL- 6, IL-7, IL-8, IL-9, IL-10, 1L-11, IL-12, IL-13, IL-14, IL-15 or IL-16.

30. VACCINE COMPOSITIONS as set forth in claims 24 to 29, **characterized in that** the compositions optionally comprise adjuvants such as CpG oligodeoxynucleotide, comprised of aluminum (aluminum hydroxide, aluminum oxide or aluminum phosphate), oily adjuvants (mineral oil, complete or incomplete Freund's adjuvants), adjuvants originating from mycolic acid (trehalose dimycolate), bacterial liposaccharides (LPS), peptidoglycans (mucopeptide), glycoproteins (N-Opaca, muramyl dipeptide analogs), proteoglycans, streptoccocal preparations (OK432), saponin, neutral oils (miglyol), vegetable oils, liposomes, polyols, vitamin E, Carbopol, interleukins and/or combinations thereof.

31. VACCINE COMPOSITIONS as set forth in claims 24 to 30, **characterized in that** the compositions also comprise pharmaceutically suitable excipients and/or carriers like liquid solutions such as saline, hypochlorite and/or other non-toxic salts, a solid solvent or a material for encapsulation where the vaccine can be resuspended or dissolved.

32. VACCINE COMPOSITIONS as set forth in claims 24 to 31, **characterized in that** the pharmaceutical excipients and vehicles further comprise amide, glucose, lactose, sucrose, gelatin, malt, rice, flower, chalk, silica gel, sodium stearate, magnesium stearate, glycerol monostearate, ascorbic acid, talc, sodium chloride, powdered milk, maltodextrin, glycerol, propylene, glycol, water, ethanol, preservatives, sweeteners and/or combinations thereof.

33. VACCINE COMPOSITIONS as set forth in claims 24 to 32, **characterized in that** the compositions comprise pharmaceutical forms such as gelatin capsules, pills, tablets, dragees, elixirs, suspensions, syrups, suspensions for vaporizers, preferably vaccines.

34. VACCINE COMPOSITIONS as set forth in claims 24 to 33, **characterized in that** the compositions comprise, particularly, gelatin capsules as carriers for lyophilized vaccines.

35. USE OF VACCINE VECTORS as set forth in claims 1 to 34, **characterized in that** it comprises the use of pharmaceutical compositions, particularly vaccines indicated for the prevention and/or treatment of infections by *Rhodococcus equi.*

36. USE as set forth in claim 35, **characterized in that** the infections by *Rhodococcus equi* comprise, particularly, pyogranulomatous pneumonia and/or colitis.

37. ANTISERA AND/OR ANTIBODIES as set forth in claims 1 to 36, **characterized in that** they are isolated or substantially purified, produced against the recombinant antigens (SEQ. ID. no. 3 and SEQ. ID. no. 4).

38. ANTISERA AND/OR ANTIBODIES as set forth in claim 37, **characterized in that** the purification comprises affinity chromatography with the recombinant antigens.

39. DIAGNOSTIC KIT as set forth in claims 1 to 38, **characterized in that** is comprises one or more recombinant Vap antigens (SEQ. ID. no. 3 and SEQ. ID. no. 4) of *R. equi* (SEQ. ID. no. 1 and SEQ. ID. no. 2) together with the specific antibodies thereof.

40. KIT as set forth in claim 39, **characterized in that** the kit comprises an immunoenzymatic assay (ELISA) for the diagnosis of infections with *R. equi.*

41. METHOD FOR PROPHYLAXIS AND/OR TREATMENT as set forth in claims 1 to 40, **characterized in that** it comprises the treatment or prophylaxis of infections by *R. equi.*

42. METHOD FOR PROPHYLAXIS AND/OR TREATMENT as set forth in claim 41, **characterized in that** the method for prophylaxis and/or treatment comprises the administration of one or more vaccine compositions, there being an initial dose and a booster dose.

43. METHOD FOR PROPHYLAXIS AND/OR TREATMENT as set forth in claims 41 and 42, **characterized in that** it comprises the administration of the initial dose on the first day of life and a booster dose fifteen days after the first dose.

44. METHOD FOR PROPHYLAXIS AND/OR TREATMENT as set forth in claims 41 to 43, **characterized in that** the administration comprises invasive methods such as parenterally, subcutaneously, intravenously, intramuscularly, intraperitoneally, transdermally, intramucously, intranasally, intragastrically and/or through non-invasive methods such as orally, rectally, by inhalation, nasally and/or ocularly, preferably orally or nasally.


LIST OF SEQUENCES

<110> USP - UNIVERSIDADE DE SÃO PAULO/ INVENT BIOTECNOLOGIA LTDA.

<120> "RECOMBINANT MICROORGANISMS AND USES THEREOF"

<130> 10.1.1748.17.5

<160> 6

<170> PatentIn version 3.5

<210> 1

<211> 519

<212> DNA

<213> Rhodococcus equi

<400> 1

atgagtgttc ggaccctttt ggcggcaacg ctcgttgctg gaatatcagt cttggcaccg 60

gccggcattg cgaacgcgga aacttcaatg gtatccacta cagcagcatc gagtgtcgag 120

cacgctgcaa acacctacga ctttgcagag gcgaagagcg ggagctctat ccccgccaaa 180

gtagccgcag agcaggcaaa cagctattcg gtccacgggc ttgtcaccag cctcgccgta 240

taccagcact tttcactgac cgttgaaggc ggcggaaaga cgtttactgg tgattctggc 300

gggatttcga ttcccggggt tgcagtgctg gagggaaccc tattcaccga ggatctgcag 360

catttgtaca gcgacaccgt ctcgttcgag tacaacgccg taggcccgta cctgaacatc 420

aacttttttg acagccatgg cactctccta ggccacgtgc agtctggatc catcgggacc 480

gtctccggca tcggtggcgg aaccggaggg tggcaatag 519

<210> 2

<211> 1111

<212> DNA

<213> Rhodococcus equi

<400> 2

atgaagactc ttcacaagac ggtttctaag gcgatcgcag ccacagccgt agctgcggct 60

gcggctatga ttcccgccgg ctgcgctaat gcgaccgttc ttgattccgg tagcagcagt 120

gcgattctca atagtggggc aggcagtggc attgtcggtt ctgggagcta tgacagctcg 180

acgacttcgt taaaccttca gaaagacgaa ccgaacggtc gagcaagcga taccgccggg 240

caagagcagc agtacgacgt tcacggagac gtcatcagcg cggtcgtcta ccagaggttt 300

cacgtattcg ggccagaagg aaaggtcttc gatggcgatg cagggggact cacgcttcct 360

ggggccggcg cgttctgggg gactctcttc acaaatgacc ttcagcgtct ctacaaagac 420

accgtctcgt tccagtacaa cgccgtgggg ccatacctga acatcaactt cttcgatagc 480

tcaggtagct tcctcggcca tatccagtcc ggtggagtta gtactgtggt gggcgtcggc 540

ggcggctctg gtagctggca caacgcctag gtcgacaaga gaggatgata tcatgagtgt 600

tcggaccctt ttggcggcaa cgctcgttgc tggaatatca gtcttggcac cggccggcat 660

tgcgaacgcg gaaacttcaa tggtatccac tacagcagca tcgagtgtcg agcacgctgc 720

aaacacctac gactttgcag aggcgaagag cgggagctct atccccgcca aagtagccgc 780

agagcaggca aacagctatt cggtccacgg gcttgtcacc agcctcgccg tataccagca 840

cttttcactg accgttgaag gcggcggaaa gacgtttact ggtgattctg gcgggatttc 900

gattcccggg gttgcagtgc tggagggaac cctattcacc gaggatctgc agcatttgta 960

cagcgacacc gtctcgttcg agtacaacgc cgtaggcccg tacctgaaca tcaacttttt 1020

tgacagccat ggcactctcc taggccacgt gcagtctgga tccatcggga ccgtctccgg 1080

catcggtggc ggaaccggag ggtggcaata g 1111

<210> 3

<211> 172

<212> PRT

<213> Rhodococcus equi

<400> 3

Met Ser Val Arg Thr Leu Leu Ala Ala Thr Leu Val Ala Gly Ile Ser

1               5                   10                  15

Val Leu Ala Pro Ala Gly Ile Ala Asn Ala Glu Thr Ser Met Val Ser

            20                  25                  30

Thr Thr Ala Ala Ser Ser Val Glu His Ala Ala Asn Thr Tyr Asp Phe

        35                  40                  45

Ala Glu Ala Lys Ser Gly Ser Ser Ile Pro Ala Lys Val Ala Ala Glu

    50                  55                  60

Gln Ala Asn Ser Tyr Ser Val His Gly Leu Val Thr Ser Leu Ala Val

65                  70                  75                  80

Tyr Gln His Phe Ser Leu Thr Val Glu Gly Gly Gly Lys Thr Phe Thr

                85                  90                  95

Gly Asp Ser Gly Gly Ile Ser Ile Pro Gly Val Ala Val Leu Glu Gly

            100                 105                 110

Thr Leu Phe Thr Glu Asp Leu Gln His Leu Tyr Ser Asp Thr Val Ser

        115                 120                 125

Phe Glu Tyr Asn Ala Val Gly Pro Tyr Leu Asn Ile Asn Phe Phe Asp

    130                 135                 140

Ser His Gly Thr Leu Leu Gly His Val Gln Ser Gly Ser Ile Gly Thr

145                 150                 155                 160

Val Ser Gly Ile Gly Gly Gly Thr Gly Gly Trp Gln

                165                 170

<210> 4

<211> 189

<212> PRT

<213> Rhodococcus equi

<400> 4

Met Lys Thr Leu His Lys Thr Val Ser Lys Ala Ile Ala Ala Thr Ala

1               5                   10                  15

Val Ala Ala Ala Ala Ala Met Ile Pro Ala Gly Cys Ala Asn Ala Thr

            20                  25                  30

Val Leu Asp Ser Gly Ser Ser Ser Ala Ile Leu Asn Ser Gly Ala Gly

        35                  40                  45

Ser Gly Ile Val Gly Ser Gly Ser Tyr Asp Ser Ser Thr Thr Ser Leu

    50                  55                  60

Asn Leu Gln Lys Asp Glu Pro Asn Gly Arg Ala Ser Asp Thr Ala Gly

65                  70                  75                  80

Gln Glu Gln Gln Tyr Asp Val His Gly Asp Val Ile Ser Ala Val Val

                85                  90                  95

Tyr Gln Arg Phe His Val Phe Gly Pro Glu Gly Lys Val Phe Asp Gly

            100                 105                 110

Asp Ala Gly Gly Leu Thr Leu Pro Gly Ala Gly Ala Phe Trp Gly Thr

        115                 120                 125

Leu Phe Thr Asn Asp Leu Gln Arg Leu Tyr Lys Asp Thr Val Ser Phe

    130                 135                 140

Gln Tyr Asn Ala Val Gly Pro Tyr Leu Asn Ile Asn Phe Phe Asp Ser

145                 150                 155                 160

Ser Gly Ser Phe Leu Gly His Ile Gln Ser Gly Gly Val Ser Thr Val

                165                 170                 175

Val Gly Val Gly Gly Gly Ser Gly Ser Trp His Asn Ala

            180                 185

<210> 5

<211> 34

<212> DNA

<213> Rhodococcus equi

<400> 5

gcggccgtcg acaagagagg atgatatcat gagt 34

<210> 6

<211> 31

<212> DNA

<213> Rhodococcus equi

<400> 6

gcgcgctgca gctattgcca ccctccggtt c 31